# EUROPEAN PATENT SPECIFICATION

(11) **EP 2 097 050 B1**
(45) Date of publication and mention of the grant of the patent: **11.02.2015**
(21) Application number: 07834644.2
(22) Date of filing: 22.10.2007
(51) Int. Cl.: A61F 5/058, B29C 33/38

(54) **A METHOD AND ARRANGEMENT FOR SHAPING A SKIN CONTACT ORTHOSIS, SUCH AS A FACIAL ORTHOSIS**
VERFAHREN UND ANORDNUNG ZUR FORMUNG EINER HAUTKONTAKTORTHESE, WIE EINER GESICHTSORTHESE
PROCÉDÉ ET DISPOSITION PERMETTANT DE FORMER UNE ORTHÈSE DE CONTACT DE PEAU, TELLE QU'UNE ORTHÈSE FACIALE

(30) Priority: 20.10.2006 EP 06076908
(43) Date of publication of application: 09.09.2009
(73) Proprietor: Academisch Ziekenhuis Maastricht, 6229 HX Maastricht (NL)
(72) Inventor: BOECKX, Willy Denis, 3210 Linden (BE); COLLA, Carlo Gilbert Jozef, 3630 Maasmechelen (BE); VAN DEN KERCKHOVE, Eric André Maria Clément, 3001 Heverlee (BE); GORISSEN, Kim Josephina, 6221 AT Maastricht (NL)
(74) Representative: Hatzmann, Martin
(86) International application number: PCT/NL2007/050506
(87) International publication number: WO 2008/048104

(56) References cited:
- EP-A- 1 249 328
- EP-A- 1 576 939
- WO-A-98/14143
- WO-A-98/30176
- WO-A-03/102876
- GB-A- 2 188 846
- GB-A- 2 339 551
- US-A- 5 280 305
- US-A1- 5 094 229
- US-A1- 5 846 464
- US-A1- 2005 151 839
- US-A1- 2006 055 943
- US-B1- 6 549 819

## Description

### FIELD AND BACKGROUND OF THE INVENTION

The invention relates to a method for shaping a skin contact orthosis, such as a facial orthosis. The invention further relates to a method for treating an anatomical deformation, e.g. in the face of an individual. The invention further relates to an arrangement for shaping a skin contact orthosis. The invention also relates to a computer program product, to an orthosis forming unit, to a data processing unit and to a skin contact orthosis.

In the art of facial reconstruction, it is known to use a rigid facial mask, from hereon referred to as a facial orthosis, to remodel the shape of the facial skin or the structures, e.g. bones, under the facial skin. The facial mask is made by taking a negative plaster mould, from the face of the individual to be treated. From the negative plaster mould, a positive tangible model of the face in its current state is made. Typically, the tangible model is made of plaster or clay. The tangible model is subsequently modified to a desired shape of the face, by manually scraping material off at suitable locations. For example, the facial mask may be used to treat hypertrophic scars, and material may be removed from the model at the locations of the hypertrophic areas in order to make a facial mask which exerts a (mild) pressure on the skin at the hypertrophic areas. Once the tangible model has a suitable shape, the mask is formed with a facial contact area complementary to the shape of the tangible model, by vacuum moulding the mask material using the tangible model as a mould. During the treatment, a gradual change of the face from its current shape into the ideal shape is obtained by scraping more material from the positive tangible model at the desired locations and again vacuum moulding a mask.

However, a disadvantage of these known facial masks is that manufacturing of the facial mask is complex and expensive because the tangible model is made and modified manually. Furthermore, during treatment a large amount of storage space is required to store the models, especially in case a large number of individuals is treated.

### SUMMARY OF THE INVENTION

It is a goal of the invention to provide a method for shaping a skin contact orthosis, such as a facial orthosis, which is less complex and expensive. Therefore, according to a first aspect of the invention, a method according to claim 1 is provided.

Such a method allows a less complex and expensive shaping of the skin contact orthosis, because data representing the current shape of the part of the body and data representing a desired shape of that part of the body are inputted to the data processing unit and the data processing unit controls the orthosis forming unit to form the contact surface of the skin contact orthosis to the surface shape. Accordingly, the need for a manual preparation of a plaster model, and the associated complexity and expenses, is obviated.

Furthermore, since the orthosis forming unit is controlled by the data processing unit, the need to store a number of plaster models is obviated as well, since the orthosis forming unit is controlled by the data processing unit to form the contact surface. Accordingly, the data processing unit can determine a shape for a specific individual and control the orthosis forming unit correspondingly.

WO 98/14143 discloses a system utilizing a scanner for scanning a patient's head, the scanned data being supplied to a milling machine which produces a positive mould or corrected mould of the patient's head, or to a fabrication apparatus for directly producing the orthosis. US 6,549,819 discloses a system employing a scanner for generating a three-dimensional image of a person's face and processing the image into a form that is appropriate for a milling program for forming a relief or mould. Neither of these documents, however, discloses measuring a pressure exerted by at least a part of the contact surface (76) of the skin contact orthosis to at least a part of the body of the individual during use of the skin contact orthosis.

According to a second aspect of the invention, an arrangement according to claim 9 is provided.

According to a third aspect of the invention, a computer program product according to claim 15 is provided.

These and other aspects of the invention will be apparent from and elucidated with reference to the embodiments described hereinafter. Specific embodiments of the invention are set forth in the dependent claims.

### BRIEF DESCRIPTION OF THE DRAWINGS

Further details, aspects and embodiments of the invention will be described, by way of example only, with reference to the drawings.
FIG. 1 schematically shows an example of an embodiment of an arrangement for shaping a skin contact orthosis according to the invention.
FIG. 2 schematically shows a sectional view of an example of an embodiment of a skin contact orthosis according to the invention.
FIG. 3 schematically shows a sectional view of an example of an embodiment of an adjustable model according to the invention.
FIG. 4 schematically shows a detailed view of the example of FIG. 3.

### DETAILED DESCRIPTION

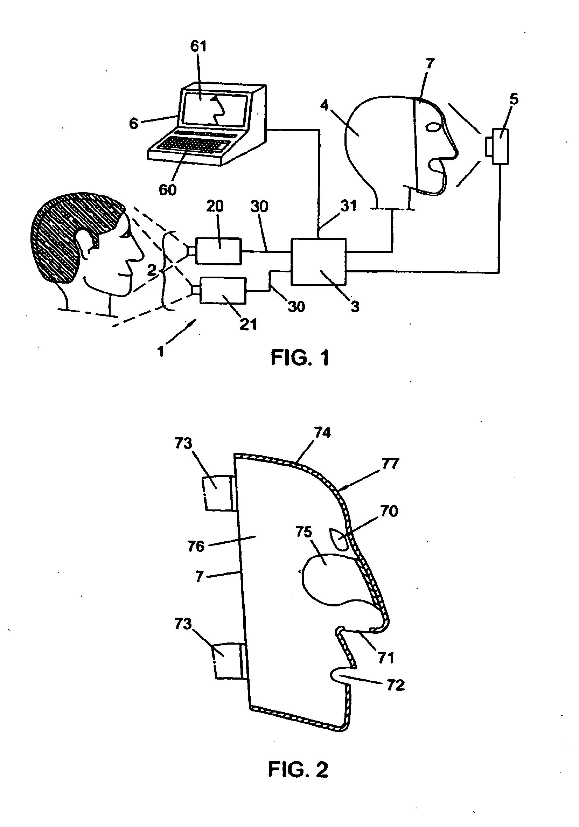

The example of an arrangement 1 shown in FIG. 1 includes a sensor unit 2, and a data processing unit 3 connected to the sensor unit 2. The sensor unit 2 can obtain information about the current shape of (at least a part of) the body, in this example (at least a part of) the face, of an individual and transmit the information to an input of the data processing unit 3, from hereon referred to as the sensor input 30.

The current shape may, for example, be determined prior to a treatment or be determined after treatment has started. The sensor unit 2 may be a contact less sensor unit which obtains the shape information without physical contact with the face. Thereby, the shaping of the orthosis is less invasive to individual.

For instance in this example, the sensor unit 2 includes an optical imaging system with a number of cameras or image capturing devices 20, 21 which are able generate a stereoscopic image of the face. The current shape of the face can be determined by the processing unit 3 from the stereoscopic image received at the sensor input 30. It should be noted that determining the shape of an object from a stereoscopic image is known in the art of stereoscopic imaging, for example of United States patent application publication US 2005-151839 A1.

However, the sensor 2 may be implemented in a different manner. The arrangement may for example include a single optical sensor which generates an image of the face and a light source which projects a line-shape beam of light on the face and which moves over the face. From the displacement of the beam of light in the image generated by the sensors, the shape of the face can be determined, as is described in United States patent application publication US 2006-0055943. A second input of the data processing unit 3, from hereon referred to as the operator input 31, is connected to a user-interface 6, in this example a suitably programmed general purpose computer 60 provided with a display 61, at which the information about the current shape of the part of the body, in this example the face, can be outputted in a for humans perceptible form. An operator, e.g. an orthopaedic engineer, can perceive the information and based thereon determine a desired shape of the part of the body, in this example the face or a part of the face. The operator may subsequently input via the user interface 6 to the data processing unit 3 information about a desired shape of the part of the body, in this example the face. Based on the data inputted by the operator, the data processing unit 3 controls the shape to which the skin contact orthosis, in this example the facial orthosis 7, is formed by an orthosis forming unit 4.

It will be appreciated that the operator, and the user interface 6, need not be present at the location where the current shape of the face is determined by the sensor unit 2. The information about the current shape of the face may, for instance, be transmitted from the sensor unit 2 to the user interface 6, e.g. via the internet. This provides the advantage that it is possible that one operator may, if so desired, process information about current shapes of faces from a plurality of sensor units which may be located at various different locations, without requiring the operator to travel to these different locations. The sensor unit may also be arranged to transmit information about the current shape of the face to a user interface of a plurality of user interfaces, e.g. depending on operator workload or expertise.

It will also be appreciated that the operator, and the user interface 6, need not be present at the location where the forming unit 4 is present. A signal representing the shape to which the facial orthosis is to be formed may, for instance, be transmitted from the user interface 6 to the forming unit 4, e.g. via the internet. This provides the advantage that it is possible that the manufacture of the orthosis is performed at a location that may be chosen independent of the location of the user interface or the sensor unit. Further, a single forming unit 4 may be arranged to, if so desired, receive signals representing the shapes to which the facial orthoses are to be formed from a plurality of user interfaces so that manufacture of orthoses can be performed centralised for a plurality of user interfaces, thus minimising production costs. The user interface may also be arranged to transmit the signals representing the shape to which the facial orthosis is to be formed to a forming unit of a plurality of forming units, e.g. if a large manufacturing capacity is required for that user interface.

It will be appreciated that the processing unit 3 may be present at the location of the sensor unit 2, at the location of the user interface 6, at the location of the forming unit 4, at a remote location or distributed among two or more of these locations.
It should be noted that the desired shape may be the desired final shape of the part of the body, in this example the face, after treatment, which from hereon is referred to as the *'ideal shape',* or the desired shape after a non-final stage of treatment, from hereon referred to as the *'intermediate shape'.* For example, the operator may input at certain times during the treatment information about an intermediate shape deemed suitable by the operator to gradually transform the shape of the face from the current shape to the ideal shape, for instance after the shape of the face at that time, i.e. after a certain period of treatment, is determined from facial data obtained with the sensor.

However, it is also possible that the operator inputs the ideal shape and the data processing unit 3 determines from the differences between the ideal shape and the current shape intermediate shapes suitable to gradually transform the face from the current shape to the ideal shape.

The operator may input at the user interface 6 parameter values related to a desired shape of at least a part of the face of the individual. The operator may input information which defines the shape directly, e.g. as coordinates in a three-dimensional space. For example, the data processing unit 3 may generate a three dimensional computer model of the current shape of the face from the facial data and output at the user interface 6 a two dimensional projection of the model. The operator may then from the projection determine a suitable desired shape. The operator may for example adjust the shape defined by the model by inputting at the user-interface suitable commands. However, the operator may also input information which defines the shape indirectly, for example in terms of the amount and location of pressure to be exerted by the orthosis on the facial skin the operator deems appropriate to bring the face into the desired shape. In such case, the data processing unit 3 may be provided with a mathematical algorithm which, based on e.g. the material parameters of the tangible model and the elastic parameters of the skin, determines a shape of the tangible model suitable to exert the given amount of pressure at the desired location.

The orthosis forming unit 4 may be implemented in any manner suitable to form the skin contact orthosis, and for example include an adjustable tangible model of the desired shape of the face. The tangible model may for example be a negative, tangible model of the face which is used as a mould to shape the facial orthosis 7. For example, the facial orthosis 7 can be pressed on the mould with an outer surface 77 of the orthosis (which faces away from the contact surface 76 of the orthosis) oriented towards the negative model. In the example of FIG. 1, the orthosis forming unit 4 is a positive, adjustable, tangible model of the face, on which a facial orthosis 7 can be positioned. The facial orthosis has, as shown in FIG. 2, an at least partially adjustable contact surface 76 for being in use positioned against the facial skin of an individual having an anatomical deformation in the face. The contact surface 76 is positioned towards the positive tangible model. By exerting on the facial orthosis 7 a force towards the positive model, the contact surface 76 of the facial orthosis 7 will shaped into a suitable form. More in particular, the contact surface 76 will conform to the contour of the part of surface 45 of the model against which the contact surface 76 lies and hence obtain a suitable shape. In this example, the orthosis forming unit 4 exerts a force on the facial orthosis 7 by means of vacuum, however, the force may also be exerted by exerting a pressure force on the facial orthosis 7, e.g. by pressing towards the model on a non-contact surface 77 of the orthosis 7.

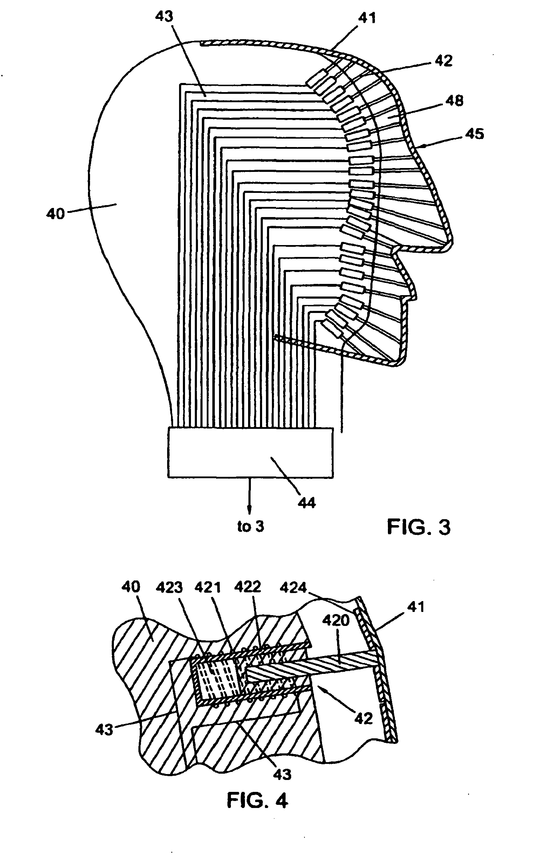

As shown in FIGs. 3 and 4, the tangible model may have a flexible surface 45 which determines the shape of the contact surface 76 of the orthosis 7 and may therefore be referred to as the orthosis forming surface 45. By adjusting the shape of the contact surface 45, for example as described below with reference to FIGs. 3 and 4, the facial orthosis 7 can be shaped into a suitable form, without the necessity of making a new model for each patient or having to adjust a plaster model by removing material. Hence, the facial orthosis can be manufactured in a less complex and expensive manner. Furthermore, the tangible model can be adjusted to the face of a particular individual, and hence the need to store a model for each individual is obviated. If so desired, information defining the current and/or ideal shape of the face of an individual may be stored, e.g. in a computer memory of the processing unit 3.

In the example of FIG. 1, the surface 45 of the orthosis forming unit 4 is controlled by the data processing unit 3 to follow a contour which is a positive of the desired shape of the contact surface 76 of the orthosis 7. As shown in FIG. 3, the orthosis forming unit 4 may for example include a core or base 40 which is provided at a side with a flexible membrane 41. The membrane 41 can be formed into a suitable shape. In FIGs. 3 and 4 for example, the membrane 41 can be formed into the desired shape by varying the spacing between the core or base 40 and the flexible membrane 41. The membrane 41 can be moved towards or away from the core or base 40 by means of a plurality of movable spacers 42. The movable spacers 42 can be controlled separately by the data processing unit 3. In FIG. 3, for instance, the movable spacers 42 are connected with control lines 43 to a routing device 44 which routes control signals from the data processing unit 3 to a suitable one of the control lines 43.

The orthosis forming unit 4 in the example of FIG. 3 may further include a suitable force exerting unit. For example, the membrane 41 may be provided with small perforations or suction holes, and a pumping unit may be connected to the space 48 held by the spacers 42 between the flexible membrane 45 and the core 40, to generate a vacuum (or at least a low pressure in the space 48 which is lower than the outside pressure). The vacuum or low pressure is then transferred into suction via the suction holes and a facial orthosis 7 positioned on the orthosis forming unit 4 will be sucked onto the orthosis forming unit 4 and be forced to conform to the shape of the flexible membrane 45 and hence be formed into the desired shape.

In the example of fig, 3 and 4, an arrangement of spacers 420 is provided. As shown in more detail in FIG. 4, the controllable spacers 42 may include a shaft 420 which is movable relative to the core 40 and which supports the flexible membrane 45 at a supporting end 424 which lies adjacent to and in contact with the flexible membrane 45. Thus, when the shaft is moved to a position, the flexible membrane 45 flexes correspondingly, and when the shaft is kept in position, the flexible membrane maintains shape. Accordingly, by moving the spacers 420 such that their supporting ends 424 are positioned corresponding to the desired shape, the shape of the model can be adjusted. In the example of FIG. 4, the shaft 420 is provided at the protruding end 424 with a plate-shape tile which, in this example, extends substantially perpendicular to the longitudinal axis of the shaft 420 and supports the flexible membrane over a larger area. The supporting ends 424 of the arrangement hence form a pattern of supporting tiles each of which can be moved in a direction perpendicular to the respective tile. The tiles and/or shafts may be oriented such that the arrangement itself follows a general facial contour and by moving the shafts 420 the shape can be adjusted to the shape of the face of a specific individual.

The movement of the shaft 420 may be driven and the position of the shafts be controlled in any manner suitable for the specific implementation. The shaft 420 may for instance be part of a linear motor. In FIG. 4, for instance, the shaft 420 is part of a magnetic linear motor. The shaft 420 can slide in and out of a recess 423 and is made of a magnetic material. Electro-magnets 421 are positioned near the recess 423. A magnetic field can be generated in the recess 423 by applying a current to the electromagnet 421, e.g. via the control line 43. By means of the magnetic field, the magnetic shaft 420 can be driven in or out of the recess 423 in a controllable manner.

FIG. 2 shows an example of a skin contact orthosis 7, in this example a facial orthosis. The example includes a mask 74 which has an inner surface 76 defining a cavity. During treatment, the mask 74 will be positioned on the face of an individual such that the face of the individual to be treated is placed in the cavity with the inner surface 76 positioned in contact with and lying against at least a part of the facial skin. The mask 74 is provided with suitable passages 70-72 for e.g. the mouth, nose and eyes of the individual. The mask 74 can be held in position with elastic bands 73 which may extend from a side of the mask 74 along the head to another side of the head.

The mask 74 may be of a material with a stiffness at room temperature sufficient to exert a pressure onto at least a part of the face. Without whishing to be bound to any theory, it is found that the skin is sensitive to pressure and that by exerting, during a prolonged time, a pressure the structure of the skin can be remodelled. A suitable period of time has been found to be between about 12 and 24 months, as an average a period of 16 months has been found to be suitable. In particular, it is found that the thickness of scars, such as hypertrophic scars caused by burns, trauma or surgical incisions can be reduced by exerting pressure.

The material of the mask 74 may be a thermoplastic material which softens sufficiently upon heating to adjust the shape of the mask. Hence, the mask of the thermoplastic material is sufficiently stiff to exert a pressure onto at least a part of the face when at room or body temperature, and the mask of the thermoplastic material is sufficiently soft to be allow adjustment of the shape of the mask when at elevated temperature. For example, the thermoplastic material may soften sufficiently to be formed by vacuum moulding. Thereby, the life time of the facial orthosis can be prolonged, since the facial orthosis can be adjusted during treatment in a simple manner, e.g. in the example of FIG. 1 by controlling the shape of the flexible membrane to an adjusted shape and forming the facial orthosis on the tangible model. Hence in a simple manner the need to manufacture a new mask any time an adjusted shape is required is obviated. Further, since the mask only needs to be heated for adjusting the shape, no hazardous materials need be used which improves safety. The mask 74 may be pre-formed prior to forming the mask 74 to the desired shape with the arrangement 1, and for example be formed to a general face-like contour. The mask 74 may for example be of a sheet-like material which is formed in a suitable three dimensional shape by the arrangement 1 (and may optionally be pre-formed).

The material of the mask 74 may be gas-permeable, and optionally liquid permeable, and/or be transparent to visible light. In the example of FIG. 2, the mask 74 is made of a gas and liquid permeable material and the inner surface 76 is locally covered with a patch 75 of a material which is gas-permeable but liquid and vapor-impermeable, e.g. silicone or an other occlusive material. Without whishing to be bound to any theory, this allows the skin to breath, while increasing the amount of moisture in the skin. Thereby, the height and/or structure of scars can be reduced. A further reduction of the height and/or improvement of the structure of scars can be obtained by exerting at the location of the patch 75 a (mild) pressure by means of the mask 74. A suitable amount of pressure is found to be a pressure of equal to or larger than 25 mm Hg.

In addition, it is also possible that the skin contact orthosis includes a drug delivery system which provides a suitable type of drug to the scarred area. For example, the surface of the mask may be provided with a coating which releases a drug. Also, a fluid supply structure mouthing near the patch may be provided, which during use provides a fluid which contains a suitable drug.

The arrangement of FIG. 1 may be used to treat an anatomical deformation in the face of an individual. In this respect, an anatomical deformation may for example include a deformation of a type requiring medical supervision, such as severe scars, burns, surgical incisions and post traumatic skin lesions, scars and deformations with cosmetic deformations. Accordingly, the treatment may be a medical treatment or a cosmetic treatment. Without whishing to be bound to any theory, a water impermeable, gas permeable membrane, such as the patch 75 in the example of FIG. 2, is believed to restore moisture in the skin, plumping indentations and smooth the appearance since it acts as the epidermis. Furthermore, it is believed that covering the facial skin at least partially with a water impermeable, gas permeable membrane results in an increased production of good quality collagen and elastin and increases the aesthetic appearance of the skin by improving its texture. Accordingly, the facial orthosis may be used in a cosmetic treatment.

During treatment, the facial orthosis 7 is placed on the face of the individual to be treated in such a manner that the facial orthosis 7 exerts the desired type of influence on the facial skin and/or bone structure. For example, the facial orthosis 7 may (locally) exert a pressure e.g. to remodel the bone structure or to reduce the formation of hypotrophic scars. However, the orthosis may also influence the face in another manner and for example (at least partially) inhibit the transport of moisture away from the skin surface or release compounds which e.g. influence the face or relieve pain, or any other suitable type of influence. After a suitable period of use of the skin contact orthosis by the individual, an adjusted skin contact orthosis may then be provided with a contact surface having an adjusted shape, for example to ensure that the amount of exerted pressure remains sufficient to remodel the bone and/or to treat the skin. The adjusted facial orthosis may for example be obtained by adjusting the current facial orthosis or by manufacturing a new orthosis with an adjusted shape.

In the foregoing it was already described that exerting a pressure to at least a part of the body by means of the mask 74 may provide a further reduction of the height and/or improvement of the structure of scars. During the progress of the method for treating the anatomical deformation, the height of a scar reduces. This causes the mask to make more even contact with the face over a larger area. Thus, the contact pressure at the position of the scar will reduce when the height of the scar reduces. Reduction of the contact pressure at the scar makes the method less effective. As already described, a pressure of equal to or larger than 25 mm Hg is preferred for effective treatment of the anatomical deformation.

In an advanced embodiment of a method for shaping a skin contact orthosis and/or treating an anatomical deformation using the mask 74 it is of benefit to measure the pressure exerted by the mask in use, e.g. continuously or at intervals, during the treatment. Thereto a pressure sensor, or a plurality of pressure sensors, may be placed between the mask and the face. When a plurality of sensors is used the respective pressures exerted to each individual pressure sensor may be measured e.g. simultaneously or sequentially. Preferably a sheet-shaped pressure sensor is used between the mask and the face. Those skilled in the art will appreciate that it is possible to calculate a change in the pressure exerted by the mask due to insertion of the pressure sensor. It will be appreciated that the pressure sensor may be an integrated part of the mask.

The measurement of the pressure exerted by the mask can be used to determine whether or not the adjusted facial orthosis should be provided or the current facial orthosis suffices. If a measured pressure at a predetermined location between the face and the mask is below a predetermined threshold pressure, e.g. below 15 mm Hg, preferably below 25 mm Hg, it may be decided to provide the adjusted facial orthosis. Thus it is possible to objectively determine whether the current facial orthosis is properly acting on the skin, or whether the adjusted facial orthosis should be provided. Thus, it is possible to recognize in an early stage that the effectivity of the facial orthosis is decreasing, if the measured pressure is close to or below the predetermined threshold pressure. Hence, it is possible to ensure that at all times the effetiviy of the facial orthosis is guaranteed. This allows a reduction of the time needed to implement the desired correction to the face.

Measuring and checking the pressure at at least one location between the orthosis and the body may also be performed automatically, e.g. by a verification unit. The verification unit is thereto arranged to receive at least one pressure measurement signal from at least one pressure sensor between the orthosis and the body, compare the measured pressure associated with the received pressure measurement signal with the predetermined threshold pressure and determine, on the basis of the comparison whether an adjusted orthosis should be provided, and optionally indicate this e.g. to the operator. In an advanced embodiment, the verification unit estimates, on the basis of a pressure measurement, when the pressure exerted by the orthosis to the body, at that location, will become smaller than the threshold pressure: The verification unit may estimate, on the basis of at least two pressure measurements, taken at the same location between the orthosis and the body, but at different moments in time, when the pressure exerted by the orthosis to the body, at that location, will become smaller than the threshold pressure. Thus, the verification unit may estimate when the adjusted orthosis will be required. This provides the advantage that the adjusted orthosis may be manufactured beforehand, so that the adjusted orthosis is already present when needed, or that an appointment may be planned beforehand to have the current orthosis adjusted.

In another advanced embodiment the data processing unit 3 is arranged to determine from the pressure measurement and the ideal shape, and optionally the current shape, an intermediate shape of the orthosis suitable to gradually transform the face from the current shape to the ideal shape.

In the foregoing specification, the invention has been described with reference to specific examples of embodiments of the invention. It will, however, be evident that various modifications and changes may be made therein without departing from the scope of the invention as set forth in the appended claims. For example, the facial mask 74 may be provided with more than one patch 75 and the patch may be at other locations than shown in the example. Furthermore, the adjustable tangible model 4 may be implemented in another manner and e.g. form a part of an injection mould or otherwise.

In the examples a facial orthosis has been described. It will be appreciated that a method for shaping a skin contact orthosis and/or treating an anatomical deformation according to the invention can also be applied to other body parts. A skin contact orthosis can also be used e.g. to treat an anatomical deformation, such as severe scars, burns, surgical incisions and post traumatic skin lesions, scars and deformations with cosmetic deformations, on the hand of an individual

The method of the invention may also be implemented in a computer program for running on a computer system, at least including code portions for performing steps of a method according to the invention when run on a programmable apparatus, such as a computer system or enabling a programmable apparatus to perform functions of a device or system according to the invention. Such a computer program may be provided on a data carrier, such as a CD-rom or diskette, stored with data loadable in a memory of a computer system, the data representing the computer program. The data carrier may further be a data connection, such as a telephone cable or a wireless connection.

Also, the invention is not limited to physical devices or units implemented in non-programmable hardware but can also be applied in programmable devices or units able to perform the desired device functions by operating in accordance with suitable program code. Furthermore, the devices may be physically distributed over a number of locations, while functionally operating as a single arrangement. For example, in the example of FIG. 1, the data processing unit 3 may be connected with the sensor input 30 to a source of data, i.e. the sensors 2, at a location different from the location to which the operator input 31 is connected. For example, the sensors 2 may be present at the practice of a physician treating the individual, such as a plastic surgeon, burn surgeon, dermatologist, orthopaedic surgeon or otherwise, and the user interface 6 may be present in the working space of an orthopaedic engineer.

Also, devices functionally forming separate devices may be integrated in a single physical device. For example, in the example of FIG. 1 the data processing unit 3 may be included in the general purpose computer 60.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to advantage.

## Claims

1. A method for shaping a skin contact orthosis (74), such as a facial orthosis, including a contact surface (76) for being in use positioned against the skin of at least a part of the body of an individual having an anatomical deformation in that part of the body, the method including:
inputting to a data processing unit (3) shape data about a current shape of at least that part of the body of said individual;
inputting to the data processing unit input data representing parameter values related to a desired shape of at least that part of the body of the individual;
determining by the data processing unit, from the shape data and the input data, a surface shape of said contact surface;
forming the contact surface of the skin contact orthosis by an orthosis forming unit (4) to the surface shape under control of the data processing unit;
**characterised in that** the method further includes:
measuring a pressure exerted by at least a part of the contact surface (76) of the skin contact orthosis to at least a part of the body of the individual during use of the skin contact orthosis; and
adjusting the skin contact orthosis, or providing an adjusted skin contact orthosis, with a contact surface having an adjusted shape when the measured pressure is smaller than a predetermined threshold pressure.

2. A method according to claim 1, further including:
estimating, on the basis of the measured pressure, at what moment in time the pressure exerted by at least that part of the contact surface (76) of the skin contact orthosis (74) to at least that part of the body of the individual during use of the skin contact orthosis will be smaller than a predetermined threshold pressure.

3. A method according to any one of the preceding claims, further including:
determining by the data processing unit (3), from the shape data and the measured pressure an intermediate shape suitable to gradually transform the part of the body from the current shape to the desired shape.

4. A method according to any one of claims 1-3, wherein a sensor for measuring the pressure is placed between the skin contact orthosis (74) and the skin.

5. A method according to any one of claims 1-3, wherein a sensor for measuring the pressure is an integrated part of the skin contact orthosis (74).

6. A method according to any one of the preceding claims, wherein:
the orthosis forming unit (4) includes an adjustable tangible model, such as a mould; the method further including adjusting the tangible model under control of the data processing unit (3), such as adjusting a mould surface of the mould to have said model shape to have a model shape corresponding to the desired shape; and
said contact surface of the skin contact orthosis is formed, e.g. by moulding the skin contact orthosis with the mould, in accordance with the model shape.

7. A method according to any one of the preceding claims, wherein said skin contact orthosis (74) has an at least partially adjustable part, e.g. made of a thermoplastic material, which defines said contact surface, and wherein forming said contact surface may include heating at least said adjustable part to a temperature sufficient to form the thermoplastic material and forming the heated thermoplastic material to the desired shape.

8. A method according to any one of the preceding claims, further including:
determining by the data processing unit (3), from the shape data and the input data, at least two surface shapes of said contact surface, wherein at least one of said surface shapes represents an intermediate shape suitable to gradually transform the part of the body from the current shape to the desired shape.

9. An arrangement (1) for shaping a skin contact orthosis (74), such as a facial orthosis, including a contact surface (76) for being in use positioned against the skin, e.g. the facial skin, of at least a part of the body, e.g. the face, of an individual having an anatomical deformation in that part of the body, said arrangement including:
a first input for receiving shape data about a current shape of that part of the body of said individual;
a second input for receiving input data representing parameter values related to a desired shape of at least that part of the body of the individual;
a data processing unit (3) connected to said first input and said second input, for determining from the shape data and the input data a surface shape of said contact surface of the skin contact orthosis; and
an orthosis forming unit (4) for forming the contact surface of the skin contact orthosis, said orthosis forming unit including a control input connected to the data processing unit, for setting the orthosis forming unit by the data processing unit in accordance with the suitable shape;
**characterised in that** the arrangement further comprises a sensor arranged to determine a pressure exerted by at least a part of the contact surface of the skin contact orthosis to at least a part of the body of the individual during use of the skin contact orthosis,
wherein the data processing unit (3) is arranged for indicating that a skin contact orthosis with the contact surface having an adjusted shape should be provided when the measured pressure is smaller than a predetermined threshold pressure.

10. Arrangement (1) according to claim 9, wherein the data processing unit (3) is arranged for estimating, on the basis of the measured pressure, at what moment in time the pressure exerted by at least that part of the contact surface (76) of the skin contact orthosis (74) to at least that part of the body of the individual during use of the skin contact orthosis will be smaller than a predetermined threshold pressure.

11. An arrangement (1) according to any one of claims 9-10, wherein the sensor is positioned at a location remote from the user interface and/or the terminal is positioned at a location remote from the orthosis forming unit.

12. An arrangement (1) according to any one of claims 9-11, wherein the sensor is communicatively connected or connectable to the user interface via a communications link, such as the internet, and/or the user interface is communicatively connected or connectable to the orthosis forming unit via a communications link, such as the internet.

13. An arrangement according to any one of claims 9-12, wherein the sensor in use is placed between the skin contact orthosis (74) and the skin.

14. An arrangement according to any one of claims 9-12, wherein the sensor is an integrated part of the skin contact orthosis (74).

15. A computer program product including program code for performing a method when run on a programmable apparatus, said method including:
receiving shape data about a current shape of at least a part of a body of an individual;
receiving data representing parameter values related to a desired shape of at least that part of the body of the individual; determining from the shape data and the input data, a suitable shape of a contact surface (76) of a skin contact orthosis (74), such as a facial orthosis, including the contact surface for being in use positioned against the skin, e.g. the facial skin, of at least a part of the body, e.g. the face, of an individual having an anatomical deformation in that part of the body;
controlling an orthosis forming unit (4) to form a contact surface of a skin contact orthosis in accordance with the suitable shape;
determining a pressure exerted by at least a part of the contact surface of the skin contact orthosis to at least a part of the body of the individual during use of the skin contact orthosis; and
determining an adjusted shape of the contact surface when the measured pressure is smaller than a predetermined threshold pressure.

## Patentansprüche

1. Verfahren zur Formung einer Hautkontaktorthese (74) wie einer Gesichtsorthese, umfassend eine Kontaktoberfläche (76), die bei Verwendung gegen die Haut von mindestens einem Körperteil einer Person mit einer anatomischen Deformation in diesem Körperteil positioniert wird, wobei das Verfahren Folgendes umfasst:
die Eingabe von Formdaten über eine aktuelle Form von mindestens diesem Körperteil der Person in eine Datenverarbeitungsvorrichtung (3);
die Eingabe von Eingabedaten, die Parameterwerte bezogen auf eine gewünschte Form von mindestens diesem Körperteil der Person darstellen, in die Datenverarbeitungsvorrichtung;
das Bestimmen einer Oberflächenform der Kontaktoberfläche anhand der Formdaten und der Eingabedaten durch die Datenverarbeitungsvorrichtung;
das Formen der Kontaktoberfläche der Hautkontaktorthese durch eine Ortheseformungsvorrichtung (4) zur Oberflächenform unter der Steuerung der Datenverarbeitungsvorrichtung;
**dadurch gekennzeichnet, dass** das Verfahren ferner umfasst:
das Messen eines Drucks, ausgeübt von mindestens einem Teil der Kontaktoberfläche (76) der Hautkontaktorthese auf mindestens einen Körperteil der Person während der Verwendung der Hautkontaktorthese; und
das Anpassen der Hautkontaktorthese oder das Bereitstellen einer angepassten Hautkontaktorthese mit einer Kontaktoberfläche, die eine angepasste Form hat, wenn der gemessene Druck kleiner als ein vorbestimmter Schwellenwertdruck ist.

2. Verfahren nach Anspruch 1, ferner umfassend:
das Schätzen, basierend auf dem gemessenen Druck, zu welchem Zeitpunkt der Druck, der von mindestens diesem Teil der Kontaktoberfläche (76) der Hautkontaktorthese (74) auf mindesten diesen Körperteil der Person während der Verwendung der Hautkontaktorthese ausgeübt wird, kleiner als ein vorbestimmter Schwellenwertdruck sein wird.

3. Verfahren nach einem der vorhergehenden Ansprüche, ferner umfassend:
das Bestimmen anhand der Formdaten und des gemessenen Drucks einer Zwischenform, geeignet zum allmählichen Transformieren des Körperteils von der aktuellen Form in die gewünschte Form, durch die Datenverarbeitungsvorrichtung (3).

4. Verfahren nach einem der Ansprüche 1-3, wobei ein Sensor zum Messen des Drucks zwischen der Hautkontaktorthese (74) und der Haut angebracht wird.

5. Verfahren nach einem der Ansprüche 1-3, wobei ein Sensor zum Messen des Drucks ein integrierter Teil der Hautkontaktorthese (74) ist.

6. Verfahren nach einem der vorhergehenden Ansprüche, wobei:
die Ortheseformungsvorrichtung (4) ein anpassbares greifbares Modell wie eine Form umfasst; das Verfahren ferner das Anpassen des greifbaren Modells unter der Steuerung der Datenverarbeitungsvorrichtung (3) umfasst, wie das Anpassen einer Formoberfläche der Form, um die Modellform zu haben, um eine Modellform entsprechend der gewünschten Form zu haben; und
die Kontaktoberfläche der Hautkontaktorthese beispielsweise durch Formung der Hautkontaktorthese mit der Form entsprechend der Modellform geformt wird.

7. Verfahren nach einem der vorhergehenden Ansprüche, wobei die Hautkontaktorthese (74) ein mindestens teilweise anpassbares Teil, beispielsweise hergestellt aus einem thermoplastischen Material, aufweist, das die Kontaktoberfläche definiert, und wobei die Formung der Kontaktoberfläche das Erwärmen von mindestens dem anpassbaren Teil auf eine Temperatur, ausreichend zum Formen des thermoplastischen Materials und Formung des erwärmten thermoplastischen Materials zu der gewünschten Form, umfasst.

8. Verfahren nach einem der vorhergehenden Ansprüche, ferner umfassend:
das Bestimmen anhand der Formdaten und der Eingabedaten von mindestens zwei Oberflächenformen der Kontaktoberfläche durch die Datenverarbeitungsvorrichtung (3), wobei mindestens eine der Oberflächenformen eine Zwischenform darstellt, die geeignet ist, um den Körperteil allmählich von der aktuellen Form zu der gewünschten Form zu transformieren.

9. Anordnung (1) zur Formung einer Hautkontaktorthese (74), wie einer Gesichtsorthese, einschließlich einer Kontaktoberfläche (76) die bei Verwendung gegen die Haut positioniert wird, wie beispielsweise die Gesichtshaut von mindestens einem Körperteil, beispielsweise des Gesichts, einer Person, die in diesem Körperteil eine anatomische Deformation hat, wobei die Anordnung Folgendes umfasst:
eine erste Eingabe zum Empfangen von Formdaten über eine aktuelle Form dieses Körperteils der Person;
eine zweite Eingabe zum Empfangen von Eingabedaten, die Parameterwerte bezogen auf eine gewünschte Form von mindestens diesem Körperteil der Person darstellen;
eine Datenverarbeitungsvorrichtung (3), verbunden mit der ersten Eingabe und der zweiten Eingabe, um anhand der Formdaten und der Eingabedaten eine Oberflächenform der Kontaktoberfläche der Hautkontaktorthese zu bestimmen; und
eine Ortheseformungsvorrichtung (4) zur Formung der Kontaktoberfläche der Hautkontaktorthese, wobei die Ortheseformungsvorrichtung eine mit der Datenverarbeitungsvorrichtung verbundene Steuereingabe umfasst, um die Ortheseformungsvorrichtung durch die Datenverarbeitungsvorrichtung entsprechend der geeigneten Form einzustellen;
**dadurch gekennzeichnet, dass** die Anordnung ferner einen Sensor umfasst, der geeignet ist, einen Druck zu bestimmen, der von mindestens einem Teil der Kontaktoberfläche der Hautkontaktorthese auf mindestens einen Körperteil der Person während der Verwendung der Hautkontaktorthese ausgeübt wird,
wobei die Datenverarbeitungsvorrichtung (3) geeignet ist anzuzeigen, dass eine Hautkontaktorthese mit der Kontaktoberfläche, die eine angepasste Form hat, bereitzustellen ist, wenn der gemessene Druck kleiner als ein vorbestimmter Schwellenwertdruck ist.

10. Anordnung (1) nach Anspruch 9, wobei die Datenverarbeitungsvorrichtung (3) geeignet ist, anhand des gemessenen Drucks zu schätzen, zu welchem Zeitpunkt der Druck, ausgeübt von mindestens diesem Teil der Kontaktoberfläche (76) der Hautkontaktorthese (74) auf mindestens diesen Körperteil der Person während der Verwendung der Hautkontaktorthese, kleiner als ein vorbestimmter Schwellenwertdruck sein wird.

11. Anordnung (1) nach einem der Ansprüche 9-10, wobei der Sensor an einem von der Benutzeroberfläche entfernten Ort positioniert ist und/oder das Datenendgerät an einem von der Ortheseformungsvorrichtung entfernten Ort positioniert ist.

12. Anordnung (1) nach einem der Ansprüche 9-11, wobei der Sensor über eine Kommunikationsverbindung wie beispielsweise das Internet kommunikativ mit der Benutzeroberfläche verbunden oder mit dieser verbindbar ist und/oder die Benutzeroberfläche über eine Kommunikationsverbindung wie beispielsweise das Internet kommunikativ mit der Ortheseformungsvorrichtung verbunden oder mit dieser verbindbar ist.

13. Anordnung nach einem der Ansprüche 9-12, wobei der Sensor bei Verwendung zwischen der Hautkontaktorthese (74) und der Haut platziert wird.

14. Anordnung nach einem der Ansprüche 9-12, wobei der Sensor ein integraler Teil der Hautkontaktorthese (74) ist.

15. Computerprogrammprodukt, umfassend einen Programmcode zum Durchführen eines Verfahrens, wenn ausgeführt auf einer programmierbaren Vorrichtung, wobei das Verfahren Folgendes umfasst:
das Empfangen von Formdaten über eine aktuelle Form von mindestens einem Körperteil einer Person;
das Empfangen von Daten, die Parameterwerte, bezogen auf eine gewünschte Form von mindestens diesem Körperteil der Person, darstellen; das Bestimmen, anhand der Formdaten und der Eingabedaten, einer geeigneten Form der Kontaktoberfläche (76) einer Hautkontaktorthese (74) wie einer Gesichtsorthese, einschließlich der Kontaktoberfläche, die bei Verwendung gegen die Haut, wie beispielsweise die Gesichtshaut von mindestens einem Körperteil, wie beispielsweise dem Gesicht einer Person mit einer anatomischen Deformation in diesem Körperteil, positioniert wird;
das Steuern einer Ortheseformungsvorrichtung (4), um eine Kontaktoberfläche einer Hautkontaktorthese entsprechend der geeigneten Form zu formen;
das Bestimmen eines Drucks, ausgeübt von mindestens einem Teil der Kontaktoberfläche der Hautkontaktorthese auf mindestens einen Körperteil der Person während der Verwendung der Hautkontaktorthese; und
das Bestimmen einer angepassten Form der Kontaktoberfläche, wenn der gemessene Druck kleiner als ein vorbestimmter Schwellenwertdruck ist.

## Revendications

1. Procédé permettant de former une orthèse de contact de peau (74), telle qu'une orthèse faciale, comprenant une surface de contact (76) destinée, en service, à être positionnée contre la peau d'au moins une partie du corps d'un individu ayant une déformation anatomique dans la partie du corps en question, le procédé comprenant :
l'entrée, dans une unité de traitement de données (3), de données de forme concernant la forme actuelle d'au moins la partie en question du corps dudit individu ;
l'entrée, dans l'unité de traitement de données, de données d'entrée représentant des valeurs de paramètre liées à une forme désirée d'au moins la partie en question du corps de l'individu ;
la détermination par l'unité de traitement de données, à partir des données de forme et des données d'entrée, d'une forme de surface de ladite surface de contact ;
le formage de la surface de contact de l'orthèse de contact de peau par une unité de formage d'orthèse (4) conformément à la forme de surface sous le contrôle de l'unité de traitement de données ;
**caractérisé en ce que** le procédé comprend en outre :
la mesure d'une pression exercée par au moins une partie de la surface de contact (76) de l'orthèse de contact de peau sur au moins une partie du corps de l'individu pendant l'utilisation de l'orthèse de contact de peau ; et
l'ajustement de l'orthèse de contact de peau ou la fourniture d'une orthèse ajustée de contact de peau, avec une surface de contact ayant une forme ajustée lorsque la pression mesurée est inférieure à un seuil de pression prédéterminé.

2. Procédé selon la revendication 1, comprenant en outre :
l'estimation, sur la base de la pression mesurée, du moment auquel la pression exercée par au moins la partie en question de la surface de contact (76) de l'orthèse de contact de peau (74) sur au moins la partie en question du corps de l'individu pendant l'utilisation de l'orthèse de contact de peau sera inférieure à une pression seuil prédéterminée.

3. Procédé selon l'une quelconque des revendications précédentes, comprenant en outre :
la détermination par l'unité de traitement de données (3), à partir des données de forme et de la pression mesurée, d'une forme intermédiaire apte à transformer progressivement la partie du corps de la forme actuelle en la forme désirée.

4. Procédé selon l'une quelconque des revendications 1 à 3, dans lequel un capteur de mesure de la pression est placé entre l'orthèse de contact de peau (74) et la peau.

5. Procédé selon l'une quelconque des revendications 1 à 3, dans lequel un capteur de mesure de la pression fait partie intégrante de l'orthèse de contact de peau (74).

6. Procédé selon l'une quelconque des revendications précédentes, dans lequel :
l'unité de formage d'orthèse (4) comprend un modèle tangible ajustable, tel qu'un moule ; le procédé comprenant en outre l'ajustement du modèle tangible sous le contrôle de l'unité de traitement de données (3), tel que l'ajustement d'une surface de moule du moule pour avoir ladite forme de modèle correspondant à la forme désirée ; et
ladite surface de contact de l'orthèse de contact de peau est formée, par exemple par moulage de l'orthèse de contact de peau avec le moule, conformément à la forme du modèle.

7. Procédé selon l'une quelconque des revendications précédentes, dans lequel ladite orthèse de contact de peau (74) comporte une partie au moins partiellement ajustable, réalisée par exemple dans un matériau thermoplastique, qui définit ladite surface de contact, et dans lequel le formage de ladite surface de contact peut comprendre le chauffage d'au moins ladite partie ajustable à une température suffisante pour former le matériau thermoplastique et le formage du matériau thermoplastique chauffé pour obtenir la forme désirée.

8. Procédé selon l'une quelconque des revendications précédentes, comprenant en outre :
la détermination par l'unité de traitement de données (3), à partir des données de forme et des données d'entrée, d'au moins deux formes de surface de ladite surface de contact, dans lequel au moins une desdites formes de surface représente une forme intermédiaire apte à transformer progressivement la partie du corps de la forme actuelle en la forme désirée.

9. Agencement (1) permettant de former une orthèse de contact de peau (74), telle qu'une orthèse faciale, comprenant une surface de contact (76) destinée, en service, à être positionnée contre la peau, par exemple la peau faciale, d'au moins une partie du corps, par exemple le visage, d'un individu ayant une déformation anatomique dans la partie en question du corps, ledit agencement comprenant :
une première entrée permettant de recevoir des données de forme concernant une forme actuelle de la partie en question du corps dudit individu ;
une seconde entrée permettant de recevoir des données d'entrée représentant des valeurs de paramètre liées à une forme désirée d'au moins la partie en question du corps de l'individu ;
une unité de traitement de données (3) reliée à ladite première entrée et à ladite seconde entrée, permettant de déterminer, à partir des données de forme et des données d'entrée, une forme de surface de ladite surface de contact de l'orthèse de contact de peau ; et
une unité de formage d'orthèse (4) permettant de former la surface de contact de l'orthèse de contact de peau, ladite unité de formage d'orthèse comprenant une entrée de commande reliée à l'unité de traitement de données, permettant à l'unité de traitement de données de paramétrer l'unité de formage d'orthèse en fonction de la forme adaptée ;
**caractérisé en ce que** l'agencement comprend en outre un capteur conçu pour déterminer une pression exercée par au moins une partie de la surface de contact de l'orthèse de contact de peau sur au moins une partie du corps de l'individu pendant l'utilisation de l'orthèse de contact de peau,
dans lequel l'unité de traitement de données (3) est conçue pour indiquer qu'une orthèse de contact de peau dont la surface de contact a une forme ajustée doit être fournie lorsque la pression mesurée est inférieure à une pression seuil prédéterminée.

10. Agencement (1) selon la revendication 9, dans lequel l'unité de traitement de données (3) est conçue pour estimer, sur la base de la pression mesurée, à quel moment la pression exercée par au moins la partie en question de la surface de contact (76) de l'orthèse de contact de peau (74) sur au moins la partie en question du corps de l'individu pendant l'utilisation de l'orthèse de contact de peau sera inférieure à une pression seuil prédéterminée.

11. Agencement (1) selon l'une quelconque des revendications 9 -10, dans lequel le capteur est placé à un endroit situé à distance de l'interface utilisateur et/ou le terminal est placé à un endroit situé à distance de l'unité de formage d'orthèse.

12. Agencement (1) selon l'une quelconque des revendications 9 - 11, dans lequel le capteur est ou peut être relié de manière à communiquer avec l'interface utilisateur via une liaison de communications telle que l'Internet, et/ou l'interface utilisateur est ou peut être reliée de manière à communiquer avec l'unité de formage d'orthèse via une liaison de communications telle que l'Internet.

13. Agencement selon l'une quelconque des revendications 9 à 12, dans lequel le capteur, en service, est placé entre l'orthèse de contact de peau (74) et la peau.

14. Agencement selon l'une quelconque des revendications 9 à 12, dans lequel le capteur fait partie intégrante de l'orthèse de contact de peau (74).

15. Produit-programme informatique comprenant un code de programme permettant d'effectuer un procédé lorsqu'il est exécuté sur un dispositif programmable, ledit procédé comprenant :
la réception de données de forme concernant une forme actuelle d'au moins une partie du corps d'un individu ;
la réception de données représentant des valeurs de paramètre liées à une forme désirée d'au moins la partie en question du corps de l'individu ; la détermination, à partir des données de forme et des données d'entrée, d'une forme adaptée d'une surface de contact (76) d'une orthèse de contact de peau (74), telle qu'une orthèse faciale, comprenant la surface de contact destinée, en service, à être positionnée contre la peau, par exemple la peau faciale, d'au moins une partie du corps, par exemple le visage, d'un individu ayant une déformation anatomique dans la partie en question du corps ;
la commande d'une unité de formage d'orthèse (4) pour former une surface de contact d'une orthèse de contact de peau selon la forme adaptée ;
la détermination d'une pression exercée par au moins une partie de la surface de contact de l'orthèse de contact de peau sur au moins une partie du corps de l'individu pendant l'utilisation de l'orthèse de contact de peau ; et
la détermination d'une forme ajustée de la surface de contact lorsque la pression mesurée est inférieure à une pression seuil prédéterminée..
